(19)

Europäisches
Patentamt
European
Patent Office
Office européen
des brevets

(11)     EP 3 693 972 A1

(12)     **EUROPEAN PATENT APPLICATION**
published in accordance with Art. 153(4) EPC

(43) Date of publication:
**12.08.2020   Bulletin 2020/33**

(21) Application number: **17927953.4**

(22) Date of filing: **03.10.2017**

(51) Int Cl.:
*G16H 10/40* (2018.01)      *G16H 50/20* (2018.01)

(86) International application number:
**PCT/RU2017/000734**

(87) International publication number:
**WO 2019/070143 (11.04.2019 Gazette 2019/15)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Atlas Biomed Group Limited**
**London E1W 1DD (GB)**

(72) Inventors:
• **MUSIENKO, Sergej Vladimirovich**
**Moskovskaya obl. 141092 (RU)**
• **PERFIL'EV, Andrej Valentinovich**
**Moscow 115533 (RU)**
• **OSIPENKO, Dmitrij Aleksandrovich**
**Moscow 124498 (RU)**
• **NIKOGOSOV, Dmitrij Arkad'evich**
**Moscow 119270 (RU)**
• **ALEKSEEV, Dmitrij Glebovich**
**Moscow 127349 (RU)**
• **TYAKHT, Aleksandr Viktorovich**
**Moscow 125315 (RU)**

(74) Representative: **Papula Oy**
**P.O. Box 981**
**00101 Helsinki (FI)**

(54) **SYSTEM AND METHOD FOR INTERPRETING DATA AND PROVIDING RECOMMENDATIONS TO A USER BASED ON HIS/HER GENETIC DATA AND ON DATA RELATED TO THE COMPOSITION OF HIS/HER INTESTINAL MICROBIOTA**

(57)     This invention relates to the field of computer technology in genetics and microbiology, and more specifically, to a new system and method for researching and interpreting genetic data and data on the composition of human intestinal microbiota in the field of microbiology. A system for providing recommendations to the user based on genetic data and / or data on a composition of the intestinal microbiota comprises a primary data acquisition unit configured to obtain the user's genetic data and / or user's intestinal microbiota data; a quality control unit configured to check the quality of the user's genetic data and the user's intestinal microbiota data obtained by the primary data acquisition unit, wherein the genetic data includes single nucleotide polymorphisms, and the microbiota data includes reads; a unit for population analysis of a user configured to determine paternal and maternal haplogroups, the population composition of the user based on his/her genetic data; a unit for taxonomic analysis of microbiota data, performed with an ability to classify metagenomic risks; a disease risk determination unit configured to determine the protection against diseases, as well as mutation testing for the presence of pathogenic alleles and disease status evaluation, a trait detection unit configured to determine states of the user traits by reducing the trait dependency graph; a unit for generating recommendations to the user, configured to form a recommendation to the user based on the data of the disease risk determination unit and the trait determination unit.

The technical result is the accuracy increase of recommendations to the user based on his/her genetic data and data on the composition of the intestinal microbiota.

Fig. 1

EP 3 693 972 A1

**Description**

TECHNICAL FIELD

This invention relates generally to the field of computer technology in genetics and microbiology, and more particularly to a new system and method for studying and interpreting genetic data and / or data on a composition of human intestinal microbiota in the field of microbiology in order to make recommendations for the user.

BACKGROUND

The human body is one of the most densely populated habitats on Earth. The number of microorganisms living in such a "biological system" is about 100 trillion bacteria, which is much higher than the total number of eukaryotic cells of all human tissues and organs. Only 10% of the body's cells are its own, the remaining 90% belong to bacteria. The totality of all human microorganisms is called microflora or microbiota, and the totality of their genes is called a metagenome. At the same time, the human metagenome is 100-150 times larger than the human genome itself. Most of the microorganisms are in the gastrointestinalal tract, so its research and interpretation of these data are a very important technical problem. In fact, the idea of the intestinal (gut) microbiota as a separate organ of the human body is being formed nowadays, which does not contradict the historically formed definition of the organ as part of the organism, which is an evolutionarily developed complex of tissues united by a common function, structural organization and development. In this case, a person can be considered as a "superorganism", the metabolism of which is provided by a well-organized work of enzymes encoded not only by the genome of Homo sapiens, but also by the genomes of all microorganisms.

Human genetics is congenital trait of a human being transmitted through genes, which are parts of DNA that carry information about heredity. Human genetics often contributes to the occurrence of the most common diseases. We cannot disregard the hereditary characteristics of a human in determining the lifestyle and diet, choosing a profession, practicing some kind of sport, etc. Multifactorial diseases develop under the influence of several factors, for example, such as ecology, lifestyle, physical activity and heredity. Accordingly, we can reduce risks adjusting the modifiable factors. Thus, knowledge of genetic risks is important for the formation of individual preventive measures. Many factors cause violations of all processes in the body and carry the development of various diseases that can be prevented by examining the genetic data of a human and forming recommendations on the factors: health, nutrition, sports, and the way of life.

Considering the great influence of microbiota and genetics on human health, efforts related to their research and interpretation should be continued.

SUMMARY OF THE INVENTION

The present invention is aimed at eliminating the drawbacks inherent in solutions known in the background art.

The technical task or problem addressed in this invention is the formation of recommendations on lifestyle, disease prevention, nutrition and physical activity to the user based on genetic data and / or data on the composition of the intestinal microbiota.

The technical result of the above technical problem is to increase the accuracy of recommendations to a user based on the consideration of genetic data and data on the composition of the intestinal microbiota.

This technical result is achieved due to the implementation of a system for providing recommendations to a user based on genetic data and / or data on a composition of the intestinal microbiota which comprises a primary data acquisition unit configured to obtain genetic data and / or intestinal microbiota data from the user; a quality control unit configured to check a quality of the user's genetic data and the user's intestinal microbiota data obtained by the primary data acquisition unit, wherein the genetic data comprise single nucleotide polymorphisms, and the microbiota data comprise reads; a unit for population analysis of genetic data configured to determine paternal and maternal haplogroups, a population composition of the user's genetic data; a unit for taxonomic analysis of microbiota data configured to map metagenomic reads to a catalog consisting of a set of sequences of microbial genes of intestinal microbiota; a disease risk determination unit configured to determine disease protection, as well as mutation testing for the presence of pathogenic alleles and disease status assessment; a trait determination unit configured to determine states of the user traits by reducing a trait dependency graph; a unit for generating recommendations to the user, made with an ability to formulate recommendations to the user based on the data of the disease risk determination unit and the user trait determination unit.

The primary data acquisition unit receives sequencing files in FASTQ or FASTA format obtained from a sequencer in some embodiments of the invention.

In some embodiments of the invention, the quality control unit obtains genetic data of the user from a silicon biochip by means of a biochip scanner.

The genetic data comprises data on the genotypes of single nucleotide polymorphisms of the user, including the X and Y chromosome polymorphisms in some embodiments of the invention.

The quality control unit additionally determines the user's genetic sex by counting a number of single nucleotide polymorphisms on the X and Y chromosomes in some embodiments of the invention.

In the case of a male, the quality control unit converts single nucleotide polymorphisms in a homozygous state on the X and Y chromosomes into single nucleotide polymorphisms in a hemizygous state in some embodiments of the invention.

The quality control unit filters out reads with an average quality value obtained from the DNA sequencer below a predetermined threshold in some embodiments of the invention.

The quality control unit removes positions having a low quality value from the ends of reads in some embodiments of the invention.

The quality control unit filters out extraneous genetic information in reads not related to the intestinal microbiota having both a biological origin and technical origin arising due to reading of artefactual genetic sequences in some embodiments of the invention.

The unit for population analysis of genetic data determines a paternal haplogroup based on a mutation tree for the Y chromosome and the user's genetic data in some embodiments of the invention.

The unit for population analysis of genetic data determines a maternal haplogroup based on a mutation tree of the mitochondria and the user's genetic data in some embodiments of the invention.

The unit for population analysis of genetic data determines a population composition based on data on genotypes of people from different populations and the user's genetic data in some embodiments of the invention.

The unit for population analysis of genetic data determines a total number of Neanderthal alleles based on the user's genetic data and a set of alleles inherited from Neanderthals in certain polymorphisms in some embodiments of the invention.

The unit for taxonomic analysis of microbiota data maps metagenome reads to a catalog, wherein the catalog includes genomic sequences of bacteria and / or archaea and / or eukaryotes occurring in the user's intestine, in some embodiments of the invention.

The unit for taxonomic analysis of microbiota data determines a relative abundance of microbial genome or microbial species, in some embodiments of the invention.

The unit for taxonomic analysis of microbiota data generates reduced tables of abundance for other taxonomic levels apart from the taxonomic level of a genus or a species, in some embodiments of the invention.

The disease risk determination unit estimates an anomaly of the sample composition by checking the total percentage of reads relating to one of the taxon from the list of opportunistic pathogens or microbes not known to be associated with the intestinal microbiota, in some embodiments of the invention.

The disease risk determination unit determines a user's disease protection from the microbiota data based on the reference data, in some embodiments of the invention.

The trait determination unit performs a check for cycles in a dependency graph, and in the presence of cycles, the unit does not allow the graph to be reduced, in some embodiments of the invention.

BRIEF DESCRIPTION OF THE DRAWINGS

The features and advantages of this invention will be evident from the following detailed description and the attached drawings where:

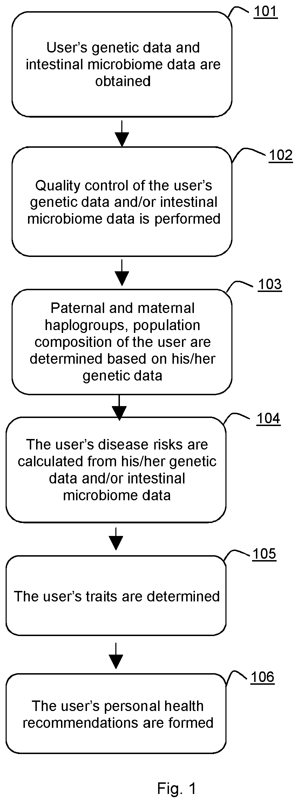

Fig. 1 - shows the block-scheme of a method for providing recommendations to a user based on genetic data and / or data on the composition of the intestinal microbiota;

Fig. 2 - shows the block-scheme of a system for providing recommendations to the user based on genetic data and / or data on the composition of the intestinal microbiota;

Fig. 3 - shows the process of the system for providing recommendations to the user based on genetic data and / or data on the composition of the intestinal microbiota;

Fig. 4 - shows an example of implementation where samples of the same users have different genotypes;

Fig. 5 - shows an example of implementation where the genotype of the same user may differ depending on the number of samples.

DETAILED DESCRIPTION OF THE INVENTION

The terms and their definitions used in the description of the invention will be considered in detail below.

In this invention, a system means a computer system, ECM (electronic computing machine), PNC (programmed numerical control), a programmable logic controller and any other devices capable of performing a specified, clearly determined operation sequence of operations (actions, instructions).

An instruction-processing device means an electronic unit or an integrated circuit (microprocessor) which executes machine instructions (programs). The instruction-processing device reads and executes machine instructions (programs) from one or more data storage devices. A data storage device can include, but not limited to hard disk drives (HDD), flash memory, ROM (read-only memory), solid state drives (SSDs), optical disk drives.

A program is a sequence of instructions to be executed by a computer control device or a command processing device.

A microbiota (normal microflora, normal flora) of a human is the totality of all microorganisms in the human body.

Genetic data is information about the DNA structure, the sequence of DNA nucleotides, single and oligonucleotide changes in the DNA sequence, including all chromosomes of a particular organism. Genetic information partially determines the morphological structure, height, development, metabolism, mental make-up, disease predisposition and genetic deficiencies of the body.

**Single nucleotide polymorphism** (SNP) is a DNA sequence of one nucleotide (A, T, G or C) in the genome (or another sequence being compared) of the same species or between homologous regions of homologous chromosomes.

**Haplogroup** is a group of similar haplotypes having a common ancestor, which had a mutation inherited by all descendants (usually single nucleotide polymorphism). The "haplogroup" term is widely used in genetic genealogy, a science that studies the genetic history of humankind, by studying the haplogroups of the Y chromosome (Y-DNA), mitochondrial DNA (mtDNA) and MHC haplogroups.

**Alleles** are different forms (values) of the same gene, located in the same areas (loci) of homologous chromosomes.

**DNA sequencing** is the determination of the sequence of nucleotides in a DNA molecule. This may be understood as amplicon sequencing (reading the sequences of isolated DNA fragments obtained as a result of a PCR reaction - such as the 16S rRNA gene or its fragments) and whole-genome sequencing (reading the sequences of the total DNA which presents in the sample).

**A homozygous state** is a state of a locus in which the alleles at the locus are identical to each other on homologous chromosomes.

**A heterozygous state** is a state of a locus in which the alleles at the locus differ from each other on homologous chromosomes.

**A hemizygous state** is a state of a locus in which there is no homologous allele, that is the chromosome in which the locus is located, does not have a homologous pair.

**RsID** is an identifier designation of an individual single nucleotide polymorphism.

**Reads** are data representing nucleotide sequences of DNA fragments obtained with a DNA sequencer.

**FASTA** is a record format of DNA sequences.

**Phylogenetics** or phylogenetic systematics is a field of biological systematics that deals with the identification and clarification of evolutionary relationships among different types of life on the Earth, both modern and extinct.

$\alpha$-**diversity** is a numerical value that characterizes the diversity of the microbial community within a single sample. $\alpha$-diversity is calculated using an algorithm based on data on species composition of the microbiota.

$\beta$-**diversity** is a numerical value characterizing the measure of the difference between the 2 microbial communities. This diversity between communities is an indicator of a differentiation degree of species distribution or the rate of change in species composition, species structure along the gradients of the environment. A possible way to determine $\beta$-diversity is to compare the species composition of different communities. The fewer common species in communities or at different points in the gradient, the higher the $\beta$-diversity.

**Mapping of short reads** is a bioinformatic method for analyzing the results of a next-generation sequencing, consisting in determining the positions in a reference base of genomes or genes wherefrom each specific short read was most likely to be received.

As a result of DNA sequencing, a set of **reads** is created. The length of a read in modern sequencers ranges from several hundred to several thousand nucleotides.

"Gold standard" (**reference**) of genome is the DNA sequence in digital form, compiled by scientists as a common representative example of the genetic code of a particular species of living organisms. In the case of the human genome, this may be, for example, the version of assembly GRChg37 (Genome Reference Consortium human genome 37), which is a haploid genome with intermittent locus (i.e., allelic variants initially listed in the same sequence may be located on different chromosomes).

**Taxonomy** - the doctrine of the principles and practice of classification and systematization of complexly organized hierarchically correlated essences.

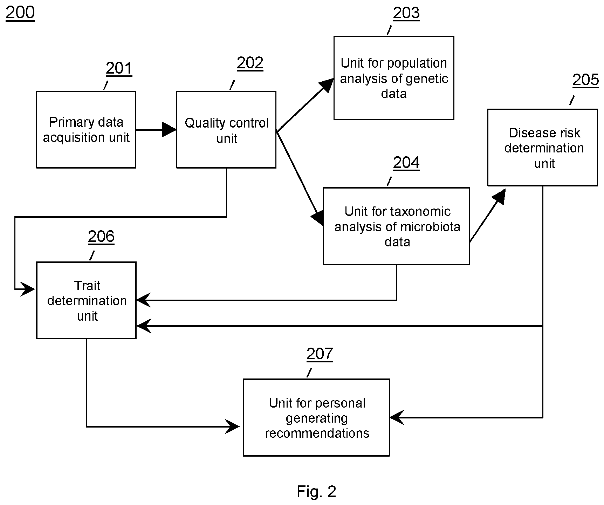

In some embodiments, a method **100** is implemented in a system **200,** which is a set of units, as shown in Fig. 2. However, the method **100** may alternatively be implemented using any other suitable system(s) configured to receive and process user genetic data and user intestinal (gut) microbiota data in conjunction with other information for creating and exchanging data obtained from microbiological analyzes.

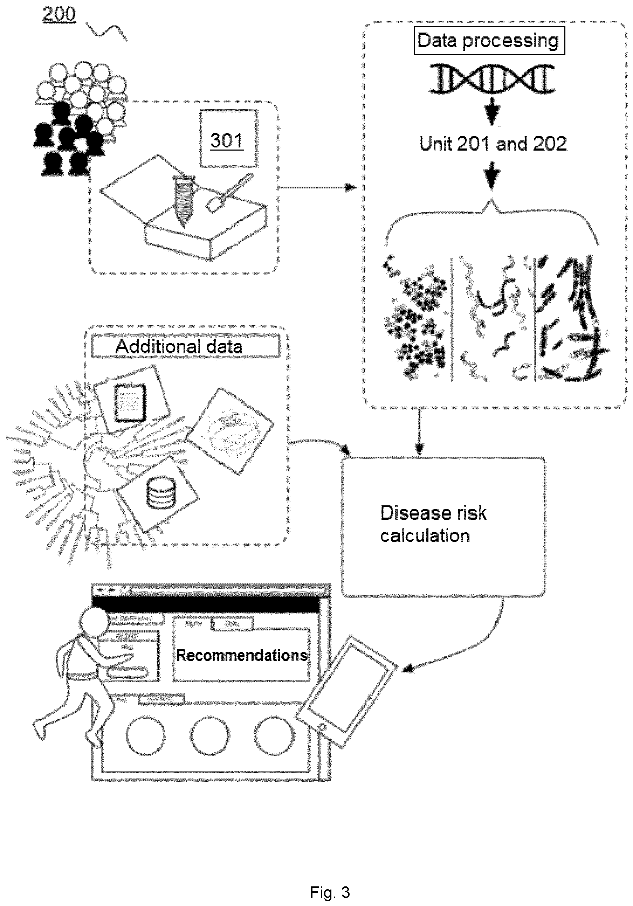

The primary data acquisition unit **201** receives samples from at least of one user. The above-mentioned data are obtained from the user by using a collection kit, including a sample container **301,** as shown in Fig. 3, having a process reagent component and configured to receive the sample from the collection point by the user. A user at a remote location from the primary data acquisition unit can provide samples in a reliable manner. Delivery of the collection kit is preferably performed using a parcel delivery service (e.g., postal service, delivery service, etc.). Additionally or alternatively, the collection kit may be provided directly through a device installed indoors or outdoors, which is intended to facilitate receiving of a sample from the user. In other embodiments, the collection kit can be submitted to a medical laboratory technician in a clinic or other medical institution. However, submitting the user's collection kit(s) to the primary data acquisition unit **201** can additionally or alternatively be performed by any other suitable method.

The kit(s) provided to the primary data acquisition unit **201** is preferably configured to facilitate collection of samples from users in a non-invasive manner. In some embodiments, non-invasive methods for obtaining a sample from a human can use any or several of the following: a permeable substrate (for example, a tampon capable of wiping a human body region, toilet paper, sponge, etc.), a container (e.g., a vial, tube, bag, etc.) configured to receive a sample from the user's body region and any other suitable element for collection (saliva, feces, urine, etc.). In a particular example the samples can be collected non-invasively from one organ or several organs, for example, such as nose, skin, human sexual organ, oral cavity and gut (e.g., using a tampon and a vial). However, the sample collection kit provided to the primary data acquisition unit **201** can additionally or alternatively be used to facilitate collection of samples in a semi-invasive manner or in an invasive manner. In some embodiments, invasive methods for receiving a sample can use the following objects: needle, syringe, biopsy magazine, trephine and any other suitable instrument for sample collection in a semi-invasive or invasive manner. In particular examples, user samples may include one or more blood samples, plasma / serum samples (for example, for extraction of cell-free DNA) and tissue samples.

Input samples can be samples (saliva, urine, feces, blood) that can be processed, for example, in a laboratory, and from which genetic data and data on the composition of the intestinal microbiota are obtained by sequencing or genotyping.

In some embodiments, the primary data acquisition unit **201** may receive additional data from sensors associated with the user(s) (e.g., sensors of portable computing devices, mobile device sensors, biometric sensors associated with the user, etc.) that will be taken into account in generating user recommendations. Thus, the primary data acquisition unit **201** can include acquiring data on a user's physical activity or physical impact on user (for example, accelerometer and gyro data from a mobile device or a user's wearable computing device), environmental data (e.g., temperature data , altitude data, climate data, light parameter data, etc.), user nutrition data or diet data (for example, data from registration records of the food received, data of spectrophotometric analysis, etc.), biometric data (e.g., data recorded by the sensors on the mobile computing device user), location data (e.g., using GPS sensors), diagnostic data, or any other suitable data. Additionally or alternatively a supplementary set of data can be obtained from the medical record and / or the clinical data of the user(s). In some embodiments, a supplementary set of data can be obtained from one or more electronic healthcare records (EHR) of the user(s).

The quality control unit **202,** based on the user's sample collection obtained in the primary data acquisition unit **201,** receives user's single nucleotide polymorphisms and reads.

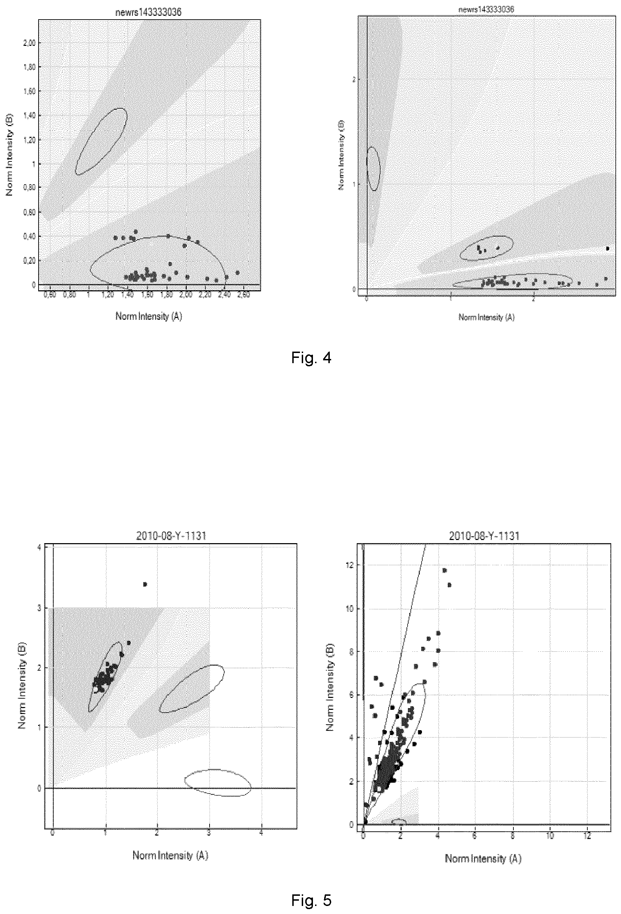

In the background art several types of errors in obtaining genetic data are known. For example, samples for the same users have different genotypes, as shown in Fig. 4. Or, for example, the genotype of the same user may be different depending on the number of samples (Fig. 5).

Earlier in the background art in order to prevent misinterpretations, geneticist implied manual check of correctness of the sample based on the number of pathogenic alleles for one single nucleotide polymorphism and the intensity indicator that is very inefficient.

During single nucleotide polymorphisms obtaining, the quality control unit **202** carries out their quality control (QC). The data can be obtained from a silicon biochip by means of a biochip scanner, which contains small pieces of DNA (probes) that specifically bind to the user's DNA. In case of successful binding a fluorescent label can be attached to this data. Biochips for genotyping allow performing SNP-typing and analysis of variations in the number of gen copies, genotyping of samples for biobanks, targeted genotyping. As a result of the operation of a biochip scanner, information about the genotypes of single nucleotide polymorphisms for a particular user is obtained, which also includes polymorphisms on the X and Y chromosomes. The above-mentioned information may include the genetic polymorphism identifier (rsID) and one or two alleles. The allele in this case is a string of A, T, G, C, - characters. For example, the data can be presented in the following form:

| # rsid | chromosome | position | genotype |
|---|---|---|---|
| rs4477212 | 1 | 72017 | A/A |
| rs3094315 | 1 | 742429 | A/A |
| rs10195681 | 2 | 8674 | C/C |
| rs11901199 | 2 | 8856 | G/G |
| rs7885198 | X | 67869221 | G |
| rs2066847 | 16 | 50729867 | -/C |

At the first stage, the user's genetic sex is determined by counting the number of single nucleotide polymorphisms on the X and Y chromosomes. In particular, the proportion of single nucleotide polymorphisms on the X chromosome in the homozygous state and the proportion of single nucleotide polymorphisms for which genotyping failed to be performed on the Y chromosome are calculated. To calculate single nucleotide polymorphisms on the X chromosome, the number of single nucleotide polymorphisms on the X chromosome in the homozygous state and the total number of single nucleotide polymorphisms on the X chromosome are determined and then the ratio of the first number to the second one is calculated. To calculate single nucleotide polymorphisms on the Y chromosome, the number of single nucleotide polymorphisms with an undefined genotype and the total number of single nucleotide polymorphisms on the Y chromosome are determined and then the ratio of the first number to the second one is calculated.

In the case of coincidence of the genetic sex determination on the X and Y chromosomes, the final genetic sex is unambiguously determined. In a case if male is defined by X and female is defined by Y, the result is X0 - a trait of Turner syndrome; -in the opposite case the result is a trait of Klinefelter syndrome. In some embodiments, in the case of a mismatch of the genetic sex determination on the X and Y chromosomes, an additional test of the sample for defect is performed, since with a high probability it is a defect, and not the two mentioned syndromes.

After the genetic sex determination by the quality control unit **202** at the quality control stage in the male case the single nucleotide polymorphisms in the homozygous state with the X and Y chromosomes are converted into single nucleotide polymorphisms in the hemizygotic state; while heterozygous single nucleotide polymorphisms on the X and Y chromosomes are filtered out and do not get into the final set of genetic data. In the female case, all single nucleotide polymorphisms on the Y chromosome are filtered out and do not get into the final set of the genetic data. Converting in this invention means a removal of one allele from a pair.

Also, the primary data acquisition unit **201** receives data by sequencing of 16S rRNA microbial genes of the intestinal microbiota. In some embodiments, the primary data acquisition unit **201** receives sequencing files in FASTQ or FASTA format from the sequencer, one file per each sample. It is preferable to use amplicon sequencing, but whole genome sequencing (WGS) may also be used.

During the sequencing, the final stage of the sequencer startup is the base calling, i.e. the conversion of intermediate "raw" (internal) signals of the device (images, spectra, intensity maps) into a plurality of reads provided with quality values (one value per each nucleotide position). Reads consist of four nucleotide characters (A, C, G and T), as well as the service character N or ".", or "?" indicating the total uncertainty in reference to a value in a given position (the sequencer cannot determine a nucleotide), for example in the following form: "GCAAAAAACTTACCCCGGAACAGGCCGAGCAGATCAAAACGCTACTGCAATACAGACCA TCAAGCACCAACTCCCNNNCGTAGNNNNNNTATGTTNNNNG". The following characteristics of reads are the most important: firstly, what length the reads will have, and secondly, what errors they can contain and how often. The device quality value is the value that characterizes the probability of error absence in a given position, calculated by the sequencer based on the signal quality:

$$Q = -10log_{10}P$$

where P is the error probability in a given position. In different embodiments, reads and their quality values can be generated as the form of two files per each sample (FASTA format) or combined into a single file (FASTQ format); while in order to save disk space, these textual representations can be converted to a binary format.

To accelerate calculations, files with the size, for example, of more than 500 MB of FASTQ format are reduced, for example, to 89951 reads (this number of reads corresponds to an average file size of 500 MB at a read length of 250 nucleotides). Beginning with a certain value, the increase of sequencing depth has little effect on the received species composition of the microbiota.

The quality control unit **202** filters out the reads with an average quality value below a predetermined threshold. In other embodiments, positions having a low quality value can be adaptively removed from the ends of reads (for example, all nucleotides from the 5 'to the 3' end are sequentially removed until a position with a quality value greater than a fixed threshold occurs). In addition, the quality control unit **202** filters out extraneous genetic information in reads having a non-biological origin, which arises due to reading of artefactual sequences caused by incorrect chemical modification of the initial DNA.

When performing the quality control process, the quality control unit **202** can use computational methods (e.g., statistical methods, machine learning methods, artificial intelligence methods, bioinformatics methods, etc.).

Then, the quality control unit **202** transmits a list of single nucleotide polymorphisms of the user with the coordinates (chromosome and its position) and the user's genotype to the unit for population analysis of genetic data **203.**

Haplogroups may be of two types: maternal haplogroup and paternal haplogroup.

In the unit for population analysis of genetic data **203,** the paternal haplogroup is first determined based on a mutation tree for the Y chromosome and the user's genetic data. The mutation tree can be represented, for example, in XML format. The genetic data of the user includes a list of single nucleotide polymorphisms with coordinates (chromosome and position) and with the user's genotype. The mutation tree for the Y chromosome comprises mutations that are characteristic for each haplogroup (position - polymorphism).

The data structure and calculation method for maternal haplogroup is the same as that of paternal haplogroup, except that the maternal haplogroup is calculated from SNP (single nucleotide polymorphisms) in the MT DNA, and the paternal haplogroup is calculated from SNP (single nucleotide polymorphism) on the Y chromosome. As a result, both the paternal and maternal haplogroups are calculated for men, and only the maternal haplogroup is calculated for women.

Each haplogroup, except the original one, has one parent haplogroup, and one or more daughter haplogroup. Each haplogroup has a finite list of determining mutations. Thus, a haplogroup tree is formed, where the edges are determined by sets of mutations.

The unit for population analysis of genetic data **203** uses a mutation tree, the genetic data of the user in determining the paternal haplogroup and operates as follows:

- determines the number of occurrences of each polymorphism in the mutation tree (for example, A123G occurs 3 times in the tree, T456C occurs 22 times in the tree);
- preserves separately the maximum possible number of occurrences of polymorphism in the tree (a number is obtained, for example, 30);
- evaluates each polymorphism according to the formula: the maximum number of occurrences of a polymorphism (determined at the previous step) minus the number of occurrences of this polymorphism in the tree. This value is the weight of the polymorphism;
- searches for a coincidence of polymorphisms between the sample (user data) and each haplogroup;
- searches for non-coincidences (mismatches) of polymorphisms between the sample (user data) and each haplogroup. In the context of this invention, a non-coincident polymorphism is a polymorphism in which the mutation is the reverse one. For example, if there is a mutation A12345C in the mutation tree, and the user has A genotype, then the unit for population analysis of genetic data **203** determines that this is not a coincidence of the polymorphism.
- if there is a mutation A12345C in the mutation tree, and the user have neither C nor A genotype, the unit for population analysis of genetic data **203** reverses the mutation to a complementary chain, and T12345G is obtained. At this step, the allele designation changes to complementary one, that is alleles change as if they were on the FWD chain and became REV.
- determines the number of coincident and non-coincident polymorphisms for each haplogroup;
- estimates the haplogroup (which is an element of the mutation tree) by the formula: the sum of weights of the coincident polymorphisms minus the sum of weights of the non-coincident polymorphisms;
- searches for a path along the tree of mutations, so that the sum of the estimates of haplogroups is maximal. The final haplogroup in this path will be the desired paternal haplogroup.

Likewise, the unit for population analysis of genetic data 203 determines the maternal haplogroup, however, based on a mutation tree for the mitochondria and the user's genetic data. mtDNA stores the tree of mutations which includes stable genetic markers (haplogroups) that are repeated in all descendants. The tree is formed as follows: markers occur during mutations and accumulate in mtDNA. There is an opportunity to trace the relation of kinship of different populations by the number of coincident markers - the more markers coincide, the closer the relationship. If the markers do not coincide after a certain mutation, it can be said when the populations dispersed.

Next, the unit for population analysis of genetic data **203** determines the population composition of the user based on data on the genotypes of people from different populations, a list of single nucleotide polymorphisms with coordinates (chromosome and position), and the user's genotype.

The unit for population analysis of genetic data **203** determines the population composition by applying the principal component method. Each genetic sample from the genome base for the populations is divided into segments consisting of a certain number of single nucleotide polymorphisms, sequentially following one another in the genome. The vector is determined by the principal component method for each segment of the sample.

Similarly, the vector is determined by the principal component method for each segment of the input sample.

Each segment of the input sample refers to a certain population as a result of comparison with the vectors defined earlier.

The proportion of population is calculated as the number of segments in the sample assigned to this population, divided by the total number of the sample segments.

In some embodiments, the main component method for decomposing a sample into a vector from 12 population components can be used, with the sample fed entirely.

In some embodiments, the unit for population analysis of genetic data **203** determines a total number of Neanderthal alleles in the sample based on the list of single nucleotide polymorphisms with coordinates (chromosome and position) and the user's genotype, a set of alleles inherited from Neanderthals in certain polymorphisms as follows: if a Neanderthal allele is in a homozygous state, then +2 to the result is added, if the Neanderthal allele is in a heterozygous state, then +1 to the result, otherwise +0. Initially, a set of alleles inherited from Neanderthals can be divided into three parts according to populations: ASN, EUR and EURASN and eventually merged into one set. Next, positions on the chromosome are transferred from 37 to 38 genome assemblies.

In some embodiments, during whole genome (WGS) profiling of the microbiota composition, the unit for taxonomic analysis of microbiota data **204** maps the metagenomic reads against a non-redundant catalog consisting of a representative set of genomes of intestinal microbes. This catalog can include genomes of bacteria, as well an archae, which can be found in user's intestine. This catalog can be developed based on large public databases, as well as automatic analysis of publications available at the background art. In some embodiments, a set of reference genomes is expanded, which allows new published genomes to be added regularly. The mapping result can be saved in a BAM file. In some embodiments, the total length of the reads mapped against the genome (the depth of coverage) is determined for each genome.

In the full genome analysis of the microbiota, the relative abundance of the genome further can be determined by the unit for taxonomic analysis of microbiota data **204** by normalizing the coverage to the genome length and the total length of the mapped reads:

$$\text{Relative abundance of a gene} = 10^{12} \times \left( \frac{\sum \text{length of mapped reads/gene length}}{\text{total length of the mapped reads of the sample}} \right)$$

In the case of analysis of the microbiota by using 16S rRNA sequencing after preprocessing, the unit for taxonomic analysis of microbiota data **204** performs quantitative taxonomic data analysis by determining to which known bacterium each read of 16S rRNA (or its fragment) belongs and how to characterize reads from unknown bacteria. The search is carried out using reference-based search strategies. The taxonomic classification rests on the basic concept of an OTU (operational taxonomic unit), i.e. a determination of a bacterial species based only on a sequence of 16S rRNA. A set of reads of the 16S rRNA gene (or its region) is compared with the representative database of the gene sequences. Each read assigned to a taxonomic unit with which it has a high degree of similarity. In the case of several coincidences, it is possible to randomly assign a read to one of these OTUs. In the database each record is a representative sequence of the corresponding OTU obtained earlier as a result of cluster analysis. While the similarity threshold can be varied, traditionally in metagenomic studies the value of 97% of similarity is used as a heuristic estimate of the degree of similarity of 16S rRNA within one bacterial species. However, this value is not absolute: on the one hand, bacteria with very different sequences of this gene can occur within the same bacterial species, on the other hand, in two different species there can be identical sequences (for example, Escherichia and Shigella).

In this embodiment of the present disclosure, other two main strategies for OTU identification known from the background art may be used: a de novo search and a hybrid approach (combining elements of template-based search and de novo search).

The sequences accumulated after 16S rRNA sequencing of the microbiota are summarized in merged databases and are phylogenetically annotated. Among the most widely used databases in the background art are Greengenes (supervised base of whole sequences of the 16S rRNA gene), SILVA (includes sequences not only of 16S, but also 18S, 23S / 28S for eukaryotes), RDP (the annotation is less unified, but the volume is higher than Greengenes).

As a result of processing a set of metagenomes in the 16S rRNA format, a relative abundance table is obtained that reflects the number of reads assigned to each taxonomic unit (OTU) from the database for each sample. A reduced table of relative abundance can be determined according to the following principle:

a. If the total number of reads for a sample for each OTU is less than a threshold value (for example, 5000), such a sample is excluded from further analysis as unsuitable terms of quality and subject to repeated sequencing.

b. If the total number of reads for a sample for each OTU is greater than or equal to a threshold value (for example, 5000), then the number of reads for each OTU is proportionally normalized so that the total number of reads for the sample becomes equal to the threshold value (for example, 5000).

In some embodiments, the relative abundance is normalized. For this purpose, the number of reads which were successfully mapped against the reference database is summarized for each sample. The normalized abundance for each taxon is calculated as the number of reads assigned to this taxon for a given sample divided by the total amount of the mapped reads for this sample and multiplied by 100%. A normalized abundance table comprising the percentage of reads assigned to each taxon from the database for each sample is obtained from the obtained values of the normalized abundance.

From unreduced tables of relative OTU abundance, the unit for taxonomic analysis **204** generates reduced abundance tables for other taxonomic levels (genera, families, etc.). For each taxonomic level, the following method is used:

a. For all OTUs that belong to a given taxonomic level, the number of reads in a sample is summarized;
b. A table of abundance is compiled from the obtained sums for this taxonomic level.

Further, based on reduced representation table (a table that reflects the number of reads assigned to each taxon at one of the taxonomic levels for each sample), the relative abundance of the groups of microbial genes is estimated.

For this, the reduced abundance table is normalized to the number of copies of 16S rRNA. For this, the number of reads assigned to each of the taxon for each sample is divided by the estimated number of copies of the 16S rRNA gene that is characteristic of a given taxon.

Then, for each gene, its abundance in each sample is determined as follows: using an existing table of the presence in different microorganisms of certain metabolic pathways and / or groups of genes involved in them, an abundance table of gene groups (EC) and metabolic pathways is compiled for each sample, which is proportional to the microorganism in which these genes / metabolic pathways are included.

As a result, the table of gene abundance in each sample is compiled from the resulting sums.

The taxonomic profile of the population of microbial communities of 16S rRNA obtained by the unit for taxonomic analysis **204** is used to evaluate important characteristics of the user's microbial population: alpha and beta diversity. They are numerical values that characterize the diversity of a single microbial community and the difference between the two communities, respectively. The more reads per sample will be sequenced, the more different species will be found, and the saturation occurs with an increase in the number of reads; it will occur faster for a community of low complexity than for a complex one; therefore, when calculating alpha diversity, the number of reads per sample is taken into account. Among the most widely used evaluators of alpha diversity, phylogenetic diversity (proportional to the fraction of the tree of life that the community covers) can be used in this invention, as well as the Chao 1 and ACE indices.

Pre-filtration of low-represented taxa is carried out, for example, according to the following principle: taxa with abundance more than 0.2% of the total microbial population in at least 10% of the samples are remained.

Further, based on the normalized abundance table contained in the unit for taxonomic analysis **204,** the disease risk determination unit **205** pre-processes and evaluates the anomalous of the microbiota composition in the sample. The total percentage of reads associated with each of taxa from the list of opportunistic pathogens is checked for each sample. A sample wherein the total percentage exceeds a fixed percentage - for example, 20% - is considered anomalous. In some embodiments, the percentage of individual taxon from the list is taken into account, including the possibility of their weighted contributions to the anomaly estimate. In some embodiments, the percentage of reads relating to the genus of bifidobacteria is checked additionally for each sample. A sample in which this percentage exceeds a fixed one - for example, 50% - is considered anomalous. In some embodiments, the relative abundance of taxon for each sample can be reviewed by an expert to detect atypical abundance of a number of taxa, including conditionally pathogenic ones. Based on the judgment of the expert and / or the results of the work of machine learning algorithms the sample can also be considered anomalous. Samples that are recognized as anomalous are excluded from further analysis. Users who own these samples are notified of an unusual microbiota composition.

Then, the disease risk determination unit **205** determines the user's disease protection using the microbiota data based on the normalized abundance table and the database of bacterial and disease links.

Preliminarily a context (a reference data for comparison) is created from the microbiota samples of a population set as follows.

For each taxon (genus or other level), a set of fixed percentiles for the abundance is calculated - for example, 33% - and 67% -percentiles. In other words, two thresholds of abundance are obtained: one-third of the samples from the population set have a smaller abundance for the given bacterium than the smaller threshold; and a third of the samples from the population set have a larger abundance for the given bacterium than the larger threshold.

In some embodiments, the threshold values for percentiles can be pre-calculated based on the results of statistical analysis of the relative abundance of the taxon in patients with this disease (or individuals at increased risk of the disease) compared to healthy individuals.

For each sample, the disease risk determination unit **205** determines user's protection against each disease. Each disease is preliminary assigned a list of microbial taxa (biomarkers) associated with it. Next, the sample is set to a disease protection value , which can be calculated according to the following rules:

For this sample, each microorganism (taxon) from a number of disease biomarkers is assigned the value 0, N (k) or M (k) (where k is the biomarker number, and N (k) and M (k) are the biomarker constants specific for the disease) according to the following rules:

> i. If a given bacterium is not contained in a given sample, this bacterium is assigned a number 0.

> ii. If the abundance of a given bacterium in the sample is lower than the upper percentile and above the lower percentile, this bacterium is assigned the number 0.

> iii. If this bacterium is not affected by this disease according to the association of bacteria and diseases, this bacterium is assigned the number 0.

> iv. If the abundance of this bacterium in this sample exceeds the upper percentile and, according to the table of bacteria and disease links, is positively associated with this disease, this bacterium is assigned the number -M (k).

> v. If the abundance of this bacterium in this sample is below the lower percentile and, according to the association of bacteria and diseases, is positively associated with this disease, this bacterium is given the number N (k).

> vi. If the abundance of this bacterium in this sample is higher than the upper percentile and, according to the association of bacteria and diseases, is negatively associated with this disease, this bacterium is assigned the number 1.

> vii. If the abundance of this bacterium in this sample is below the lower percentile and, according to the association of bacteria and diseases, is negatively associated with this disease, this bacterium is assigned the number -1.

In some exemplary embodiments, (k = 1, ...), N (k) = M (k) = 1 for all biomarkers.

The sample is assigned the disease protection value, equal to the sum of the values assigned to the biomarker bacteria in the previous step.

Fixed percentiles for protection are calculated for each disease, for example, 33% - and 67% -percentiles. In other words, two protection thresholds are obtained: a third of the samples from the population set have less disease protection than a smaller threshold; and a third of the samples from the population set have greater disease protection than the larger threshold.

Then the scaled protection value for the user is then determined by the disease risk determination unit **205** as follows:

The amount of microbiota protection is calculated by the method described above in the context analysis for each disease.

Then the user's protection is scaled according to the following rule:

> a. The lower percentile of disease protection calculated from the context is taken in the new scale for 0;

> b. The upper percentile of disease protection calculated from the context is taken in the new scale for 10;

> c. The upper percentile of disease protection calculated from the context is taken in the new scale for 10;

If the protection value on the new scale is less than 4, it is set to 4. The obtained value is the level of disease protection for the sample.

Other percentiles may be used in other embodiments of the invention. Also, each taxon may have its own individual weight, formed from an assessment of its effect on the characteristic and its representation in a particular sample, other than 1, -1, or 0.

The user recommendations propose to increase the relative abundance of bacteria that are negatively associated with the disease and have a low (non-zero) and / or normal abundance (i.e. lie between the upper and lower percentile) and if they are not positively associated with other diseases.

In some embodiments, the disease risk determination **unit 205** determines the composition of hereditary monogenic diseases. For this, a list of mutations and pathogenic alleles of hereditary diseases can be used. These data only comprise information on pathogenic mutations. The user's sample comprises the mutation identifier and genotypes.

The disease risk determination unit **205** checks each mutation for the presence of a pathogenic allele and evaluates the disease status, for example, as follows:

    a. 0 - no pathogenic allele;

    b. 1 - one and only one mutation with one pathogenic allele;

    c. 2 - one or more mutations with both pathogenic alleles;

    d. 3 - two or more mutations with one pathogenic allele (compound heterozygote);

For one disease, one sample may have the first three cases at the same time, the order of appointment: 2 > 3 > 1.

In the prior art, there are the following types of mutation inheritance: autosomal recessive (AR), autosomal dominant (AD), X-linked recessive (XR), X-linked dominant (XD), Y-linked (Y), mitochondrial (MT).

If the disease status is estimated as 2 (one or more mutations with both pathogenic alleles) or 3 (two or more mutations with one pathogen allele), the order of assigning the final inheritance type with the combination AD and AR - AD is the following AR - AD> AR; with a combination of XD and XR - XD> XR. As a result, at the output, the disease risk determination **unit 205** issues the disease status with the inheritance type.

In some embodiments, the disease risk determination unit **205** can rank users based on the obtained data (individual data obtained as a result of the disease risk calculation, as well as metagenomic analysis data). For each disease, the disease risk determination unit **205** ranks all users in terms of the relative risk ratio and divides them, for example, into five groups so that the first group includes 10% of users, the second group 20%, the third 40%, the fourth 20% , the fifth - 10%.

Further, the disease risk determination unit **205** generates, for example, the following user distribution according to the risk groups:

    1. High risk - from 0th to 10th percentile;
    2. Increased risk - from the 10th to the 30th percentile;
    3. Average risk is from the 30th to the 70th percentile;
    4. Moderate risk - from the 70th to the 90th percentile;
    5. Low risk - from 90th to 100th percentile.

Based on the results of metagenomic analysis, as was shown above, the disease risk determination unit **205** determines the degree of protection of an organism against the development of certain diseases. The protection level may be expressed in integers on a scale of 0 to 10. The disease risk determination unit **205** uses the following principles to include data on the degree of microbiota protection in the ranking of genetic risks:

•   0-5 points - the user moves to the risk group higher than the group determined by the results of the disease risk calculation (but not higher than the first group);

•   6-7 points - the risk group remains unchanged;

•   8-10 points - the user moves to the risk group lower than the group determined by the results of the disease risk calculation (but not less than the fifth one).

If the user passed only a microbiota test (genetic data are not taken into account), the distribution of risks may look like this:

    1. High risk - from 0 to 3 points;
    2. Increased risk - from 4 to 5 points;
    3. Average risk is from 6 to 7 points;
    4. Moderate risk - from 8 to 9 points;
    5. Low risk - 10 points.

It will be apparent to a person skilled in the background art that the ranking method and the points are exemplary and not restrictive and do not affect the nature of the invention.

In some embodiments, it can be assumed that all factors (external and genetic) are independent of each other in the calculation of risk. To determine the disease risk, a logistic model can be used, the starting point of which is the average occurrence of the disease in the population and the contributions of external and genetic risk factors are taken into account.

For genetic risk factors, the numerical values of the contribution can be extracted from studies such as genome-wide association study (GWAS) for this disease. For example, for such a disease as "diabetes mellitus type II" it may be the study of Morris, A.P. et al., 2012, "Large-scale association analysis provides insights into the genetic architecture and pathophysiology of type 2 diabetes. Nature Genetics", 44(9), pp.981-990.

For external risk factors, information sources are used, which shows the relationship between a particular risk factor and the risk of developing this disease. For example, the following set of factors and articles can be used for diabetes mellitus:

| risk_factor | OR | PMID |
| --- | --- | --- |
| BMI for women | BMI < 22 - 1 RR<br>BMI 22-23 - 2.9 RR<br>BMI 23-24 - 4.3 RR<br>BMI 24-25 - 5.0 RR<br>BMI 25-27 - 8.1 RR<br>BMI 27-29 - 15.8 RR<br>BMI 29-31 - 27.6 RR<br>BMI 31-33 - 40.3 RR<br>BMI 33-35 - 54.0 RR<br>BMI $\geq$ 35 - 93.2 RR | www.ncbi.nlm.nih.gov/pubmed/7872581 |
| BMI for men | BMI < 24 - 1 RR<br>BMI 24-25 - 1.6 RR<br>BMI 25-27 - 2.3 RR<br>BMI 27-29 - 4.8 RR<br>BMI 29-31 - 8.1 RR<br>BMI 31-33 - 13.8 RR<br>BMI 33-35 - 26.9 RR<br>BMI $\geq$ 35 - 50.7 RR | www.ncbi.nlm.nih.gov/pubmed/7988316 |
| Controlled drinking | 0,87 RR (Male)<br>0,60 RR (Female) | www.ncbi.nlm.nih.gov/pubmed/19875607 |
| Smoking | 1,44 RR | www.ncbi.nlm.nih.gov/pubmed/18073361 |
| Diabetes in relatives | 2,44 HR | www.ncbi.nlm.nih.gov/pubmed/23052052 |
| Presence of fresh fruit in the diet | 0,91 RR | www.ncbi.nlm.nih.gov/pubmed/26816602 |
| Presence of fresh vegetables in the diet | 0,87 RR | www.ncbi.nlm.nih.gov/pubmed/26816602 |
| Polycystic ovarian syndrome | 8,8 OR | www.ncbi.nlm.nih.gov/pubmed/24081730 |
| Presence of cereals in the diet | 0,68 RR | www.ncbi.nlm.nih.gov/pubmed/24158434 |
| Drinking coffee | 0,7 RR | www.ncbi.nlm.nih.gov/pubmed/24459154 |
| Drinking sweetened beverages | 1,26 RR | www.ncbi.nlm.nih.gov/pubmed/20693348 |

(continued)

| risk_factor | OR | PMID |
|---|---|---|
| Meat consumption 50 g / day | 1,51 RR | www.ncbi.nlm.nih.gov/pubmed/21831992 |
| Low HDL | < 1.0 mmol/L - 5,74 HR 1,0-1,4 mmol/L - 2,72 HR 1,4-1,7 mmol/L - 1,44 HR $\geq$ 1,7 mmol/L - 1,0 HR | www.ncbi.nlm.nih.gov/pubmed/25972569 |
| High level of adiponectin | 0,72 OR | www.ncbi.nlm.nih.gov/pubmed/19584347 |
| High levels of hormone-binding globulin in men | 0,12 OR | www.ncbi.nlm.nih.gov/pubmed/19657112 |
| High levels of hormone-binding globulin in women | 0,11 OR | www.ncbi.nlm.nih.gov/pubmed/19657112 |
| High CRP (above the upper limit of the reference) | 1,28 OR | www.ncbi.nlm.nih.gov/pubmed/23264288 |
| Sleep more than 9 hours or less than 6 hours a day | 1.28RR (<6 hours / day) 1.48RR (> 9 hours / day) | www.ncbi.nlm.nih.gov/pubmed/19910503 |

In some embodiments, the relative abundance of the gene groups included in the metabolic pathway of butyric acid synthesis, according to the EC nomenclature (Enzyme commission number), is determined from the composition of the microbiota sample. Their abundance correlates with contextual data, and each group of genes is assigned a point in a manner similar to that described above for calculating the disease protection. Context data on the abundance of microorganisms comprises the distribution of the abundance of prokaryotic microorganisms, values for 33% and 67% of percentiles. Then the point is determined from 4 to 10 and this will be a point associated with butyric acid synthesis. If this point is less than the threshold value, the taxa that potentially carry in genome those groups of genes are searched for because they were not represented in the first step (fell below the 33% -percentile), and their abundance by contextual data is checked. If these taxa also fall below the 33% percentile, they are used later to formulate recommendations to the user.

In other embodiments, a determining the abundance of EC gene groups in the sample forming part of the vitamin synthesis pathway, for each of the B1, B2, B3, B5, B6, B7, B9, K vitamins is performed,. Their abundance is correlated with the contextual data, each EC is assigned a point in the same way as described above. Next, the average point for all vitamins is calculated and its integer part will be a vitamin synthesis value. If this value is less than the threshold value, microorganisms that potentially possess those EC in their genome, which appeared to be underrepresented in the first step (in 33%), are searched for and their abundance by contextual data is checked. If these microorganisms also got in 33%, then they are used in the future recommendations for the user.

In some embodiments, another nomenclature of microbial functional groups of genes may be used, for example, KEGG Orthology groups or a group of genes from the MetaCyc base.

In other embodiments, the metabolizing potential is determined for each type of dietary fiber from a predetermined set. A total abundance is estimated relative to the contextual data of those microorganisms which are capable of fiber metabolism as known from the association databases. If the total abundance gets into 33%, the algorithm decides that the metabolizing potential of this fiber is low. A point of 4 to 10 is calculated for each fiber, depending on the value of their total abundance. The total fiber metabolizing potential is calculated as the integer average point for all dietary fibers.

In some embodiments, the quality control unit 202 transmits the genetic data to the user trait determination unit 206. The trait in genetic terminology is a measurable characteristic of the user. The trait can be obtained from a user-filled questionnaire, a genetic test, wearable gadgets, a medical card, etc.

Examples of user traits:

- lactose intolerance (discrete state: there is a predisposition, there is no predisposition, unknown);

- age (continuous state: 30 years, 49 years, etc.);

- CYP2D6 activity (discrete states: ultra-rapid metabolizer, a normal metabolizer, a poor metabolizer);

- risk of obesity (continuous state: risk of 50%, risk of 43.4%, etc.).

In some embodiments, the traits may be grouped into hereditary disease groups, drug reactions, nutrition symptoms, sport traits, haplotypes.

Depending on the type of the user trait, the trait can have two or more possible states. In some embodiments, the states may be discrete or continuous, but not simultaneously for the same trait. While the trait is not calculated for the user, it has an undefined state. In some embodiments, the user's trait is dependent on the states of the other traits. All possible combinations of dependencies of states form a trait definition area.

Traits can be variable (coffee consumption), invariable (CYP2D6 activity, phenylketonuria status) and conditionally variable (some risks), which depend on the variable traits.

A trait may have a limitation period, after which it will be disabled, that is, it will go into an undefined state. For example, the concentration of cholesterol in the blood test will be valid for a year, and then the characteristic will return to the state of an indeterminate user's trait.

Variable and conditionally variable traits are stored in a history of changes of their states, including disabled state after the limitation period expiration.

Since the traits may refer to other traits in their interpretation, the trait determination unit **206** forms a directed dependency graph between the traits when the system is filled with traits. The graph nodes, which do not refer to anything, are the nodes of the source data (mutations, answers to questions, microbiota). All other nodes directly or indirectly depend on the nodes of the source data.

The user trait determination unit **206** performs determination of the trait states for a particular user by reducing the graph starting from the original data nodes.

If one of the traits has changed its state, for example, the user answered the question differently in the questionnaire, all the traits dependent on the questionnaire will be recounted, i.e. updated. A recalculation of some traits will cause recalculation of the others until the end of the dependency graph is reached.

Before determining the trait state, the trait determination unit **206** checks the dependency graph for cycles, and in the presence of cycles, the unit **206** does not allow the graph to be reduced.

The trait determination unit **206** can formulate an interpretation for the user (sports, nutrition, personal qualities, etc., not limited to) based on the indicator of at least one trait, the state of the trait and the genetic data (single nucleotide polymorphism, genetic sex, etc.), for example in the following form:

| TRAIT | STATES | PREDICATE | TEXT |
| --- | --- | --- | --- |
| femaleHormoneBindingGlobulinLevels | high | and ( gender. female, "rs727428 FWD C/C" ) | You are predisposed to a high level of hormone-binding globulins. The SHBG gene variant encodes for this. Normally, the level of free estradiol can be increased. |
| femaleHormoneBindingGlobulinLevels | mean | and ( gender.female, "rs727428 FWD T/C" ) | You are predisposed to an average level of hormone-binding globulins. The SHBG gene variant encodes for this. |
| femaleHormoneBindingGlobulinLevels | low | and ( gender.female, "rs727428 FWD T/T" ) | You are predisposed to a low level of hormone-binding globulins. The SHBG gene variant encodes for this. Normally, the level of free estradiol can be reduced. |

In some embodiments, the trait determination unit **206** determines a user's trait based on the microbiota data. To do this, the results of calculating the disease protection, the metabolizing potential of dietary fibers, the synthesis of short chain fatty acids, the synthesis of vitamins, as well as a database of associations between food and intestinal microbiota are used. The database is formed using computer algorithms of text analysis in conjunction with manual addition based on facts about food products, the intake of which is positively associated with certain microorganisms living in the human intestine.

If the final point for one of the data (for example, a disease protection) is less than a predetermined threshold value, then the food products associated with the growth of those microorganisms of low abundance are taken from the database of associations. The more often a product is recommended for a given user based on the results of different algorithms, the higher its rank and the probability of its recommendation to the user.

The user recommendation generation unit **207** is configured to generate a recommendation to the user based on the data of the disease risk determination unit **205** and the user trait determination unit **206.**

Individual data obtained as a result of identifying the traits, risks and status of carriage of diseases, as well as metagenomic analysis data from other units of the system are input to the unit.

The operation of the user recommendation generation unit **207** is based on the fulfillment of the condition leading to the output of the result. The condition is a combination of simple logical operations on input data. The result is a recommendation text aimed at motivating the user to perform a specific set of activities. Recommendations in some embodiments are divided into the following groups:

- recommendations on undesirable types of physical activity;
- recommendations for lifestyle changes;
- recommendations for changing the intake of certain food or groups of food products;
- recommendations for visiting a doctor.

The group of food recommendations is given taking into account both genotyping data and data of the composition of the intestinal microbiota, or one variant.

In some embodiments, the user recommendation generation unit **207** generates risk reduction recommendations, self-diagnosis recommendations, recommendations for visiting a doctor and trait recommendations.

When formulating a recommendation for reducing the disease risk, an increased risk of the disease is a prerequisite for displaying the recommendation.

Recommendation encourages the transition from one state of the trait to another. That is, the recommendation refers to the trait itself. A trait may have an array of recommendations, the size of which is equal to the number of specified transitions between different states. The transition itself can occur only in case of the user's source data affecting the trait changes and reinterpretation is performed.

The transition may have additional conditions under which it will be occurred. For example, the user's genetic sex can influence the output of a recommendation.

The presence of a certain state of a trait defined by the trait determination unit 206 may require a certain state of another trait, i.e., there is a requester and a required state. Each of the states, among which the target one has to be chosen, has a weight that consists of the weights of all the requesters. In the event that the required state differs from the current one, the transition starts and a recommendation is issued motivating the user to make this transition. The choice of recommendation to be given to the user depends on the outweighed required state of the trait.

If the user has an increased risk of the disease, he/she is given recommendations for correcting the changed external risk factors, which he/she has in a state that increases the risk. For example, for diabetes mellitus the recommendations might look like the follows:

"Drink coffee every day.

You should include coffee in your daily diet, but not exceeding your allowable rate.

Include fruits in your daily diet.

It is recommended to eat fruits every day. They are rich in fibers, healthy vitamins and microelements.

It is recommended to eat foods rich in vitamin E.

The intake of tocopherol with food should be increased. Vitamin E is a powerful antioxidant, essential for muscle tissue and the immune system. "

In more detail, the generated recommendations in the user recommendation generation unit **207** may include providing notifications to the user about the recommended therapeutic measures and / or other options for dealing with health-related goals. Notifications of recommendations can be provided to an individual via an electronic device (for example, a personal computer, a mobile device, a tablet, a smart clock, etc.), and displayed in a graphical user interface (GUI). Recommendations can be displayed in the application, the web interface in the user's personal account, in the SMS message or PUSH-notification. In one embodiment, a web interface of a personal computer or laptop associated with a user can provide a user with access to a user account wherein the user account includes information about user data, detailed information about genetic data and data on the composition of the intestinal microbiota, and notifications of recommendations generated in the recommendation generation unit **207.** In another embodiment, an application running on a personal electronic device (e.g., smartphone, smart clock, smart head device) can be configured to provide notifications (e.g., display or sound, etc.), with respect to recommendations, obtained with the help of the recommendation generation unit **207.** Notifications may additionally or alternatively be provided directly by a person associated with the system user (e.g., caretaker, spouse, medical staff, etc.). Notifications may additionally or alternatively be provided to a person associated with the user (i.e. health service specialist), wherein the person can influence the implementation of recommendations (for example, through a prescription, a therapeutic session, etc.). However, recommendations and notifications can be provided to the user of the system in any other convenient way.

Although the embodiments are described in connection with an exemplary computing system environment, they can be implemented using numerous computing system environments, configurations, and general and special purpose devices.

Examples of known computing systems, environments and / or configurations that may be suitable for use with aspects of the invention include, but are not limited to, mobile computing devices, personal computers, server computers, handheld devices or laptops, multiprocessor systems, game consoles, systems based on microprocessors, set-top boxes, programmable consumer electronics, mobile phones, network personal computers, minicomputers, supercomputers, distributed deductions the fluids, which include any of the above systems or devices (e.g., fitness bracelets), etc. Such systems or devices can receive data from a user in any form, including input devices such as a keyboard or pointing device, through gesture input and / or via voice input.

Embodiments of the invention may be described in the general context of computer-executable instructions, such as program modules or units, executed by one or more computers or other devices. Computer-executable instructions can be organized into one or more computer-executable components or modules. Typically, program modules include, but are not limited to, subroutines, programs, objects, components, and data structures that perform particular tasks or implement particular abstract data types. Aspects of the invention can be realized by any number and any organization of such components or modules. For example, aspects of the invention are not limited to specific computer-executable instructions or specific components or modules illustrated in the figures and described herein. Other embodiments of the invention may include other computer-executable instructions or components having more or less functionality than the illustrated and described herein.

Aspects of the invention transform a general-purpose computer into a special-purpose computing system configured to interpret user genetic data and data on the composition of the intestinal microbiota.

It is to be understood that the various methods described herein may be implemented together with hardware or software, or, if necessary, with a combination thereof. Therefore, the methods and system of this subject matter, or some aspects or parts thereof, may include program code (i.e., instructions) implemented in a tangible medium such as floppy disks, CD-ROMs, hard disk drives, cloud storage, or any other storage media, wherein when the program code is loaded and executed by a machine, such as a computer, the machine becomes a device for applying the subject matter of the invention. In the case of executing program code on programmable computers, the computing device basically comprises a processor, a storage medium that is readable by the processor (including volatile and nonvolatile memory and / or memory elements), at least one input device, and at least one output device. One or more programs can implement or use the processes described with the present disclosed subject matter, for example, by using an application programming interface (API), reusable controls, and the like. Such programs can be implemented using a high-level procedural or object-oriented programming language to exchange data with a computer system. However, if necessary, the program (s) can be implemented in assembler, or machine programming language. In any case, the programming language can be a compiled or interpreted language, and it can be combined with hardware implementations.

Although the subject matter of the invention has been described by a specific language of structural features and / or methodological functions, it is understood that the subject matter of the invention has been defined in the appended claims, and it is not necessary to limit the features or functions described above. To a large extent, the features and functions described above are disclosed as exemplary embodiments of the claims.

### Claims

1. A system for providing recommendations to a user based on his/her genetic data and data on a composition of the intestinal microbiota, the system comprising:

   • a primary data acquisition unit configured to obtain genetic data and / or intestinal microbiota data from the user;
   • a quality control unit configured to check a quality of the user's genetic data and / or the user's intestinal microbiota data obtained by the primary data acquisition unit, wherein genetic data comprises single nucleotide polymorphisms and microbiota data comprises reads;
   • a unit for population analysis of genetic data configured to determine paternal and maternal haplogroups for the user, and a population composition of user's genetic data;
   • a unit for taxonomic analysis of microbiota data configured to classify reads using a database of sequences of 16S rRNA genes;
   • a disease risk determination unit configured to determine a disease risk, disease protection, to test a genotype for the presence of pathogenic alleles and to assess a status of a hereditary disease carriage;
   • a trait determination unit configured to determine states of the user traits by reducing a trait dependency graph;

• a unit for generating recommendations to the user configured to form a recommendation to the user based on the data obtained from the disease risk determination unit and the trait determination unit.

2. The system of claim 1, wherein the primary data acquisition unit receives sequencing files in FASTQ or FASTA format obtained from a sequencer.

3. The system of claim 1, wherein the quality control unit receives genetic data of the user from a silicon biochip by means of a biochip scanner.

4. The system of claim 3, wherein the genetic data comprises data on genotypes of single-nucleotide polymorphisms of the user, including the X and Y chromosome polymorphisms.

5. The system of claim 1, wherein the quality control unit further determines the user's genetic sex by counting a number of single nucleotide polymorphisms in the X and Y chromosomes.

6. The system of claim 5, wherein the quality control unit converts single nucleotide polymorphisms in a homozygous state of the X and Y chromosomes into single nucleotide polymorphisms in a hemizygous state, and wherein single nucleotide polymorphisms in a heterozygous state on the X and Y chromosomes are filtered out in the case of a male.

7. The system of claim 5, wherein all single nucleotide polymorphisms on the Y chromosome are filtered out and do not get into a final set of the genetic data in the case of a female.

8. The system of claim 1, wherein the quality control unit filters out reads with an average quality value below a predetermined threshold.

9. The system of claim 1, wherein the quality control unit removes positions having a low quality value from the end of the reads.

10. The system of claim 1, wherein the quality control unit filters out extraneous genetic information in reads of biological or non-biological origin arising due to reading of artefactual sequences.

11. The system of claim 1, wherein the unit for population analysis of genetic data determines a paternal haplogroup based on a mutation tree for the Y chromosome and the user's genetic data.

12. The system of claim 1, wherein the unit for population analysis of genetic data determines a maternal haplogroup based on a mutation tree of the mitochondria and the user's genetic data.

13. The system of claim 1, wherein the unit for population analysis of genetic data determines a population composition based on data on genotypes of people from different populations and the user's genetic data.

14. The system of claim 1, wherein the unit for population analysis of genetic data determines a total number of Neanderthal alleles based on the user's genetic data and a set of alleles inherited from Neanderthals in certain polymorphisms.

15. The system of claim 1, wherein during classification with a database of sequences of 16S rRNA genes, the database includes a set of bacterial and / or archaean genomes occurred in the user's intestine.

16. The system of claim 1, wherein the unit for taxonomic analysis of microbiota data determines a relative abundance of microbial genome or microbial species.

17. The system of claim 1, wherein the unit for taxonomic analysis of microbiota data generates reduced abundance tables for other taxonomic levels.

18. The system of claim 1, wherein the disease risk determination unit estimates an anomaly of the genetic data by checking the total percentage of reads relating to one of the taxa from the list of opportunistic pathogens.

19. The system of claim 1, wherein the disease risk determination unit determines user's disease protection from microbiota data based on reference data.

20. The system of claim 1, wherein the trait determination unit checks for cycles in a dependency graph, and in the presence of cycles disallows the graph reduction.

101

User's genetic data and
intestinal microbiome data are
obtained

102

Quality control of the user's
genetic data and/or intestinal
microbiome data is performed

103

Paternal and maternal
haplogroups, population
composition of the user are
determined based on his/her
genetic data

104

The user's disease risks are
calculated from his/her genetic
data and/or intestinal
microbiome data

105

The user's traits are determined

106

The user's personal health
recommendations are formed

Fig. 1

Fig. 2

Fig. 3

Fig. 4

Fig. 5

## INTERNATIONAL SEARCH REPORT

| International application No. |
|---|
| PCT/RU 2017/000734 |

**A. CLASSIFICATION OF SUBJECT MATTER**

*G16H 10/40 (2018.01)*
*G16H 50/20 (2018.01)*
*G06F 19/22 (2011.01)*

According to International Patent Classification (IPC) or to both national classification and IPC

**B. FIELDS SEARCHED**

Minimum documentation searched (classification system followed by classification symbols)

G16H 10/40, 50/20, G06F 19/00, 19/10, 19/22

Documentation searched other than minimum documentation to the extent that such documents are included in the fields searched

Electronic data base consulted during the international search (name of data base and, where practicable, search terms used)

DB WIPO (Patentscope), DB Espacenet, PatFT & AppFT USPTO, RUPTO, EAPATIS, CIPO, J-PlatPat, KIPRIS, K-PION, SIPO, DWPI, DEPATISnet, PSS; MedLine, PubMed Central, Scopus, EMBASE, ScienceDirect, РИНЦ (e-Library)

**C. DOCUMENTS CONSIDERED TO BE RELEVANT**

| Category* | Citation of document, with indication, where appropriate, of the relevant passages | Relevant to claim No. |
|---|---|---|
| Y | US 2016/0281166 Al (BHATTACHARJEE ARINDAM et al.) 29.09.2016, para. [0005] -[0006], [0011], [0021]-[0023], [0026], [0034], [0067], [0078], [0080], [0107]-[0122], [0125], [0132]- [0142], examples 1-3 | 1-20 |
| Y | BODILY P.M. et al. "ScaffoldScaffolder: solving contig orientation via bidirected to directed graph reduction." Bioinformatics, 2016, 32(1), p.17-24, in particular p.20-23 | 1-20 |
| Y | US 8 877442 B2 (THE BOARD OF TRUSTEES OF THE LELAND STANFORD JUNIOR UNIVERSITY) 04.11.2014, the claims | 4-7, 11, 12 |
| A | DEURENBERG RUUD H. et al. "Application of next generation sequencing in clinical microbiology and infection prevention." Journal of Biotechnology, 2017 (Available online 29 December 2016), 243, pp. 16-24, in particular abstract | 1-20 |
| A | KOSTRJUKOVA E. S. et al. "Variabelnost otnositelnogo soderzhania genomnoi DNK cheloveka pri metagenomnom analize mikrobioty kishechnika." Biomeditsinskaia khimiia, 2014, 60(6)? P./ 695-701, in particular abstract | 1-20 |

☐ Further documents are listed in the continuation of Box C.   ☐ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 29 May 2018 (29.05.2018) | 21 June 2018 (21.06.2018) |

| Name and mailing address of the ISA/ RU | Authorized officer |
|---|---|
| Facsimile No. | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MORRIS, A.P. et al.** Large-scale association analysis provides insights into the genetic architecture and pathophysiology of type 2 diabetes. *Nature Genetics,* 2012, vol. 44 (9), 981-990